# NEUE EUROPÄISCHE PATENTSCHRIFT

(11) **EP 0 415 142 B2**
(45) Veröffentlichungstag und Bekanntmachung des Hinweises auf die Entscheidung über den Einspruch: **03.09.1997**
(45) Hinweis auf die Patenterteilung: 28.12.1994
(21) Anmeldenummer: 90115284.3
(22) Anmeldetag: 09.08.1990
(51) Int. Cl.: C11D 1/94, A61K 7/50, A61K 7/48

(54) **Duschgel**
Shower gel
Gel de douche

(30) Priorität: 31.08.1989 DE 3928773
(43) Veröffentlichungstag der Anmeldung: 06.03.1991
(73) Patentinhaber: Joh. A. Benckiser GmbH, D-67059 Ludwigshafen (DE)
(72) Erfinder: Baust, Heinrich, D-6831 Plankstadt (DE); Gross, Wolfgang, D-6704 Mutterstadt (DE)
(74) Vertreter: Grussdorf, Jürgen, Dr.

(56) Entgegenhaltungen:
- EP-A- 0 127 580
- EP-A- 0 194 097
- GB-A- 2 013 235
- US-A- 3 950 417
- H.P. Fiedler, Lexikon der Hilfsstoffe für Pharmazie, Kosmetik und angrenzende Gebiete, 3. Aufl. (1989), S. 618-619
- K. Schrader, Grundlagen und Rezepturen der Kosmetika (1979), S. 86-87, 380-382, 447, 450
- K. Schrader, Grundlagen und Rezepturen der Kosmetika, 2. Aufl. (1989), S. 598-600, 690

## Beschreibung

Die Erfindung betrifft ein neues Duschgel, welches die Hautrauhigkeit vermindert.

Duschgele sind in den mannigfachsten Zusammensetzungen im Handel oder in vielen Publikationen beschrieben. Der Trend der Bevölkerung geht dazu, häufiger zu duschen, wodurch Hautirritationen. welche sich bis zu Allergien steigern können, aufgrund der in den Duschgelen enthaltenen Stoffe neuerdings vermehrt auftreten. Irritationen dieser Art werden in erster Linie durch die in den Duschgelen enthaltenen Tenside selbst hervorgerufen, insbesondere haben sich die aufgrund ihrer guten Schaumbildung besonders häufig eingesetzten Alkylsulfate und Alkylpolyoxiethylensulfate als stark hautreizend herausgestellt.

Neuerdings bestehen weiterhin Befürchtungen, daß Tenside, welche Polyoxyethylen-Strukturen enthalten, von ihrer Herstellung her spurenweise mit Dioxan bzw. Ethylenoxid verunreinigt sind und diese Produkte wiederum hautreizend wirken können.

Es stellte sich daher die Aufgabe, ein neues Duschgel zu entwickeln, welches äußert hautverträglich ist, keine pharmakologisch bedenklichen Bestandteile enthält, jedoch in Bezug auf die Schaumbildung (Schaumhöhe, Schaummenge und Anschäumverhalten) sowie die Viskosität und Temperaturbeständigkeit mit den bekannten Duschgelen zumindest vergleichbar ist.

Überraschenderweise konnte dieses Problem dadurch gelöst werden, daß als Tensidgemisch eine Kombination welche nur aus einem Amidopropylbetain und einem Fettsäuremethyltaurid besteht verwendet wird, während die übrigen Bestandteile bekannter Duschgele, beispielsweise Verdickungsmittel, Elektrolytsalze, Parfüm, Farbstoffe, Desinfektionsmittel, Eiweißhydrolysate etc. beibehalten werden.

Beide Tensidgruppen sind für ihre Einsetzbarkeit als Tenside in Badepräparaten. Shampoos. Schaumbädern und Duschbädern bekannt. Aus der EP-A1-194097 ist ein Reinigungsschaum bekannt, welcher aus einer Kombination anionischer und amphoterer Netzmittel, hautglattenden Mitteln sowie Glycerin und Wasser mittels eines Treibmittels hergestellt wird. Neben dem bevorzugten Alkylglycerinethersulfonaten kann die Mischung auch Methylacyltaurate oder amphotere Betaine enthalten. Die fraglichen Mischungen erweisen sich als wenig hautirritierend und erreichen durch die beigefügten Polymeren eine gewisse Hautglättung. Eine verdickende Wirkung wurde dabei nicht beobachtet.

Überraschenderweise hat sich nunmehr gezeigt, daß die Kombination dieser beiden Produktgruppen Amidopropylbetaine und Methyltaurid) inDuschgelen nicht nur Produkte ergibt, die eine hervorragende Schaumbildungs- und Beständigkeit aufweisen und deshalb als Waschmittel den bisher bekannten Duschgelen vergleichbar bzw. überlegen sind, sondern daß diese Mittel auch äußert hautschonend sind und nicht nur keine Irritationen hervorrufen, sondern im Gegenteil die Hautrauhigkeit im Vergleich zu einer Behandlung mit reinem Wasser oder Lösungen der beiden Komponenten alleine vermindern.

Die erfindungsgemäßen Duschgele weisen vorzugsweise die folgende Zusammensetzung auf: Duschgel, enthaltend
5 - 15 % Amidopropylbetain
2 - 10 % Fettsäuremethyltaurid
0 - 2 % Rückfetter
0 - 2 % Eiweißhydrolysat
0 - 2 % Parfüm
0 - 1 % Farbstoffe
0 - 5 % Elektrolytsalze
0 - 2 % Verdickungsmittel
0 -0,5 % Desinfektionsmittel
Rest Wasser.

Unter Amidoproylbetain im Sinne der Erfindung fallen Verbindungen der folgenden allgemeinen Formel 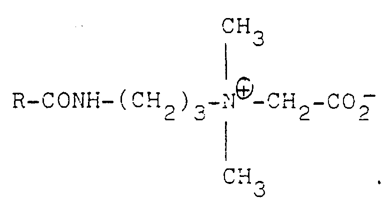 in der R eine lineare Alkylgruppe mit 7-17 C-Atomen bedeutet.

Fettsäuremethyltauride im Sinne der Erfindung entsprechen der allgemeinen Formel 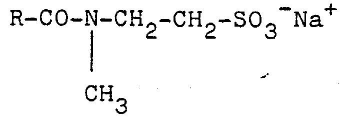 in der R einen gesättigten oder 1-2fach ungesättigten Fettsäurerest mit 8-20 C-Atomen bedeutet.

Außer diesen für das Waschverhalten und die Verdickung essentiellen Tensiden, welche in einer Gesamtmenge von 7-25 % zugefügt werden, können die erfindungsgemäßen Duschgele noch alle üblichen für diesen Zweck gebräuchlichen Hilfsstoffe enthalten. Besonders zu nennen sind in diesem Zusammenhang rückfettende Stoffe und Eiweißhydrolysate, welche die Struktur der Haut positiv beeinflussen, Parfüms und Farbstoffe, welche die Attraktivität des Produktes für den Verbraucher erhöhen, Elektrolytsalze und Verdickungsmittel, die die Viskosität des Gels zusammen mit den Tensiden bewirken, Desinfektionsmittel, die die Stabilität des Produktes bei der Lagerung einerseits und die antibakterielle Wirkung des Duschgels bei der Benutzung andererseits erzielen.

Als Lösungsmittel wird entmineralisiertes Wasser verwendet, wobei in kleinen Mengen auch pharmakologisch verträgliche, wasserlösliche organische Lösungsmittel, beispielsweise Glycerin oder ähnliches enthalten sein können.

In den folgenden Beispielen wird die Erfindung näher erläutert, ohne daß sie dadurch in irgendeiner Weise beschränkt werden soll.

### Beispiel 1

| Rohstoffe Bezeichnung | Gew.% |
|---|---|
| Amidopropylbetain¹⁾ | 9,42 |
| Fettsäure(C7-C17)methyltaurid²⁾ | 2,58 |
| Collagenhydrolysat 100% | 0,30 |
| Kathon CG® | 0,08 |
| Parfumöl | 1,0 |
| Farbstoffe | q.s. |
| Leitungswasser | ad 100 |
| Produktdaten: Viskosität ca. 5000 mPas pH conc 6,9+0,1 | |

| | |
|---|---|
| 1) Tego Betain HS® | |
| 2) Hostapon CT® | |

### Versuchsprotokoll

### Messung der Hautrauhigkeit nach DIN 4768

Die Prüfung der Hautrauhigkeit erfolgte an den Unterarm-Innenseiten von 10 Probanden mit einer 2 %igen Produktlösung gegenüber destilliertem Wasser als Referenz.

Von den Testfeldern sowie dem unbehandelt bleibenden Leerfeld wurden zunächst Silikonabdrücke angefertigt. Objektträger werden dünn mit einer farblosen, mit Härter gemischten Silikonmasse bestrichen und mit einem Strichbalken auf gleichmäßige Dicke aufgestrichen. Dann wurden die Objektträger mit konstantem Druck auf die Haut aufgelegt und nach 2 Min. abgezogen.

Für die Untersuchung wird die zu untersuchende Lösung bei 22°C Raumtemperatur auf die zu behandelnde Stelle aufgebracht, 10 Min. ohne mechanische Einwirkung eintrocknen gelassen, mit Wasser vorsichtig abgespült und getrocknet. Danach wurden erneut Silikonabdrücke gefertigt.

Nach 24 stündigem Aushärten werden alle Abdrücke mit dem Oberflächenmeßgerät Perthometer PRK ausgewertet. Mittelwerte der Rauhtiefe R_{z} nach DIN 4768 in um sind in der anliegenden Tabelle wiedergegeben.

**Tabelle**

| Mittelwerte in µm | | |
|---|---|---|
| | Leerfeld | Präp. 1550 |
| Leerwert | 37,1 | 36,2 |
| nach 10 Min. Anwendung | 37,8 | 33,2 |
| Differenzen | + 0,7 | -3,0 |

## Patentansprüche

1. Duschgel, enthaltend ein Tensidgemisch und andere übliche Hilfsstoffe, wie Verdickungsmittel, Elektrolytsalze. Parfüm, Farbstoffe, Desinfektionsmittel, Eiweißhydrolysate sowie Wasser als Lösungsmittel. dadurch gekennzeichnet, daß als Tensidgemisch eine Kombination welche nur aus einem Amidopropylbetain und einem Fettsäuremethyltaurid besteht verwendet wird.

2. Duschgel gemäß Anspruch 1, enthaltend
5 - 15 % Amidopropylbetain
2 - 10 % Fettsäuremethyltaurid
0 - 2 % Rückfetter
0 - 2 % Eiweißhydrolysat
0 - 2 % Parfüm
0 - 1 % Farbstoffe
0 - 5 % Elektrolytsalze
0 - 2 % Verdickungsmittel
0 -0,5 % Desinfektionjmittel
Rest Wasser.

3. Duschgel gemäß Anspruch 1 oder 2, dadurch gekennzeichnet, daß das Amidopropylbetain die allgemeinen Formel 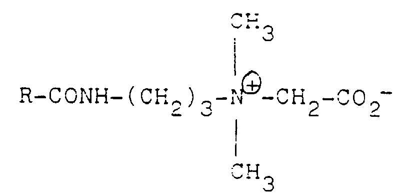 in der R eine lineare Alkylgruppe mit 7-17 C-Atomen bedeutet
und das Fettsäuremethyltaurid die Formel 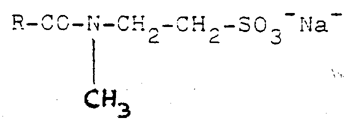 in der R einen gesättigten oder 1-2fach ungesättigten Fettsäurerest mit 8-20 C-Atomen bedeutet, hat.

## Claims

1. Shower gel containing a tenside mixture and other usual adjuvants, such as thickening agents, electrolyte salts, perfume, colouring materials, disinfection agents, protein hydrolysates, as well as water as solvent, characterised in that, as tenside mixture there is used a combination consisting only of an amidopropylbetaine and a fatty acid methyltauride.

2. Shower gel according to claim 1, containing:
5 - 15% amidopropylbetaine
2 - 10% fatty acid methyltauride
0 - 2% refattening agent
0 - 2% protein hydrolysate
0 - 2% perfume
0 - 1% colouring materials
0 - 5% electrolyte salts
0 - 2% thickening agent
0 - 0.5% disinfection agent
remainder water.

3. Shower gel according to claim 1 or 2. characterised in that the amidopropylbetaine has the general formula 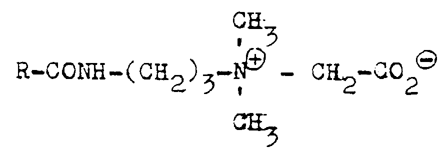 in which R signifies a linear alkyl group with 7 - 17 C-atoms, and the fatty acid methyltauride has the formula 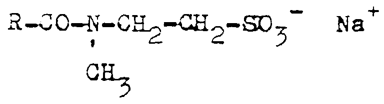 in which R signifies a saturated or 1 - 2 times unsaturated fatty acid residue with 8 - 20 C-atoms.

## Revendications

1. Gel pour douche, contenant un mélange d'agents tensioactifs et d'autres adjuvants habituels tels que des épaississants, des sels électrolytiques, des parfums, des colorants, des désinfectants, des hydrolysats de protéine, ainsi que de l'eau comme solvant, caractérisé en ce que, comme mélange d'agents tensioactifs on utilise en combinaisons consistant uniquement en une amidopropylbétaïne et en un méthyltauride d'acides gras.

2. Gel pour douche selon la revendication 1 contenant
5 - 15% d'amidopropylbétaïne
2 - 10% de méthyltauride d'acides gras
0 - 2% de reconstituant du gras
0 - 2% d'hydrolysat de protéine
0 - 2% de parfum
0 - 1% de colorant
0 - 5% de sels électrolytiques
0 - 2% d'épaississant
0 - 0,5% de désinfectant,
le reste étant de l'eau.

3. Gel pour douche selon la revendication 1 ou 2, caractérisé en ce que l'amidopropylbétaïne répond à la formule générale 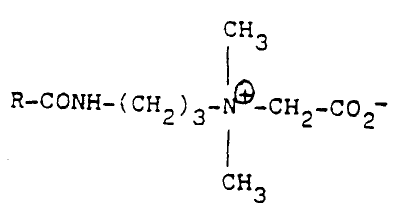 dans laquelle R représente un groupe alkyle linéaire contenant de 7 à 17 atomes de carbone.
et le méthyltauride d'acides gras répond à la formule 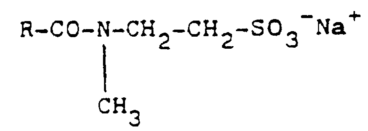 dans laquelle R représente un radical d'acides gras saturé ou 1-2 fois insaturé contenant 8-20 atomes de carbone.
